Europäisches Patentamt

European Patent Office

Office européen des brevets

(11) Publication number: **0 519 683 A1**

# EUROPEAN PATENT APPLICATION

(21) Application number: **92305508.1**

(22) Date of filing: **16.06.92**

(51) Int. Cl.⁵: **C07H 23/00**, C12Q 1/40,
C07H 15/203, C07H 3/06

(30) Priority: **17.06.91 GB 9112989**

(43) Date of publication of application:
**23.12.92 Bulletin 92/52**

(84) Designated Contracting States:
**AT BE CH DE DK ES FR GB GR IT LI LU MC
NL PT SE**

(71) Applicant: **GENZYME LIMITED (formerly
known as Genzyme (UK) Ltd)
37 Hollands Road
Haverhill, Suffolk CB9 8PU(GB)**

(72) Inventor: **Jackson, Christopher B.
33 Bockhill Road
Bury St Edmunds, Suffolk(GB)**
Inventor: **Siddique, Farhat K.
8 Colne Valley Road
Haverhill, Suffolk(GB)**
Inventor: **Stevens, David R.
10 Withersfield Road
Haverhill, Suffolk(GB)**
Inventor: **Moore, Stephen P.
6 Wickham Place
The Square, Lenham(GB)**

(74) Representative: **Froud, Clive et al
Elkington and Fife Prospect House 8
Pembroke Road
Sevenoaks, Kent TN13 1XR(GB)**

(54) **Preparation of oligosaccharide derivatives.**

(57) A process for the direct silylation of the terminal,
non-reducing glucose unit of an oligosaccharide containing at least three repeating glucose units and
having an aglycone unit at the reducing end thereof,
the aglycone being selected from nitrophenyl, 2-
chloro-4-nitrophenyl, 4-methylumbeliferone, luciferin
and chromogenic phenols, which comprises:
(a) forming a solution of the oligosaccharide in a
solvent:
(b) reacting the oligosaccharide with a silylating
protective agent in the presence of a base; and
(c) recovering a monosilylated oligosaccharide
derivative from the reaction mixture is disclosed.

This invention relates to the preparation of oligosaccharide derivatives; more particularly, it relates to the preparation and purification of 6-0-thexyldimethylsilyl-p-nitrophenyl-α-D-maltoheptaoside, which may be used as a substrate for the assay of α-amylase.

The measurement of α-amylase in urine and serum is widely performed for the differential diagnosis of abdominal disease where elevated levels indicate a pancreatic disorder, such as pancreatitis. Known methods for the determination of α-amylase employ oligosaccharide substrates which are cleaved into smaller fragments by the action of the enzyme. These fragments themselves may be determined by methods of sugar analysis or may be subject to the activity of a second exo-enzyme, such as α-glucosidase, β-glucosidase or glucoamylase, to release either further sugar molecules or a chromogen which will allow the activity to be estimated from the generation of a colour.

The prevention of substrate cleavage by the exo-enzyme incorporated into the assay prior to introduction of a sample containing α-amylase, an endo-enzyme, has been achieved by the addition of a blocking substituent on the non-reducing end of the substrate. Compounds of this type are described in US-A-4,709,020 by Raucher et al and in US-A-4,649,108 by Blair, who additionally described the use of glucoamylase in such an assay so as to enhance the performance.

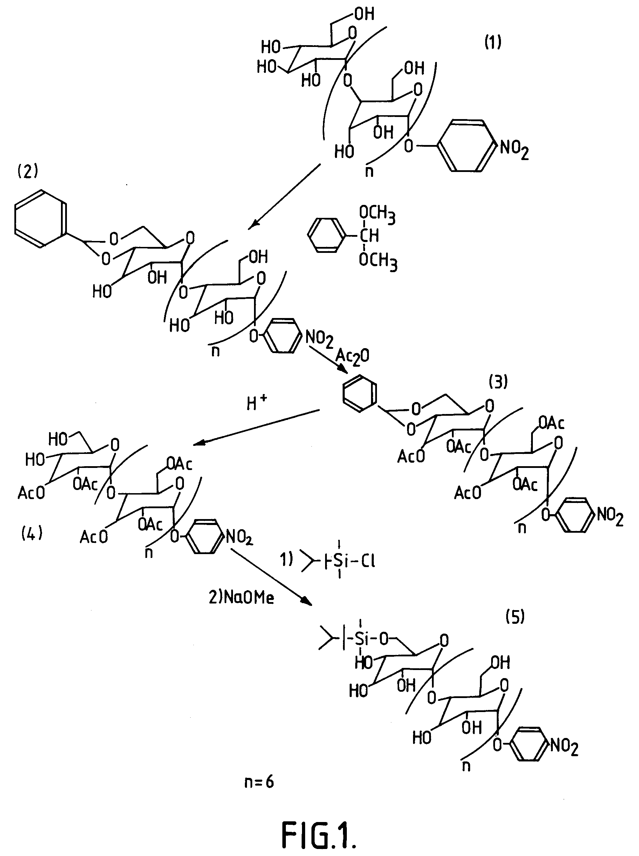

Such oligosaccharides, the preparation thereof by generally conventional means and the use thereof as improved substrates for the assay of α-amylase have been described in EP-A-452067. There is described therein the synthesis of 6-0-thexyldimethylsilyl-p-nitrophenyl-α-D-maltoheptaoside (5) by a five stage procedure starting from p-nitrophenyl-4,6-0-benzylidene-α-D-maltoheptaoside (2). (See accompanying Figure 1.)

The starting material for the five step synthesis, p-nitrophenyl-α-D-maltoheptaoside, may, for example, be obtained using either a chemical route, which may be based on that of Burns et al (GB-A-1,570,152) or an enzymic reaction using glucanotransferase (French et al, JACS, 76, 2987-2990, (1954)).

With reference to the accompanying illustrative Figure 1, the steps are as follows:

(1) Preparation of p-nitrophenyl-4,6-0-benzylidene-α-D-maltoheptaoside (2) from p-nitrophenyl-α-D-maltoheptaoside (1).

(2) Conversion of (2) to peracetylated-p-nitrophenyl-4,6-0-benzylidene-α-D-maltoheptaoside (3).

(3) Removal of the benzylidene protecting group in (3) to produce peracetylated-4,6-dihydroxy-p-nitrophenyl-α-D-maltoheptaoside (4).

(4) Conversion of (4) to peracetylated-6-0-thexyldimethylsilyl-p-nitrophenyl-α-D-maltoheptaoside.

(5) Removal of the acetate groups therefrom to give the required 6-0-thexyldimethylsilyl-p-nitrophenyl-α-D-maltoheptaoside (5).

This five step procedure has several disadvantages. The use of protecting groups leads to a lengthy multi-step process. High costs are incurred due to the time associated with carrying out this process. Isolation of the intermediates is problematical due to difficult filtrations and mechanical losses which inevitably occur when a considerable amount of material handling is required. Costs of multi-step chemical plant processes are well known to be high, thus shorter, simpler processes are always of considerable interest.

In general terms, the present invention relates to an advantageous preparation which surprisingly converts (1) directly to (5) in a single step. The present invention further relates to the purification of (5) from a mixture of silylated p-nitrophenyl-α-D-maltoheptaosides wherein (5) is the single largest silylated species. Such a purification is required to obtain the compound at very high purity, but by a procedure that is suitable for commercial use as regards both sufficient scale and limited number of steps. By way of illustration, the purification of (5) from crude product wherein it is present at less than 20% w/w to achieve a chemical purity of 99% at a process yield of 45% is central to the utility of the direct silylation reaction. Furthermore, the loading of 300 g of crude mixture of silylated p-nitrophenyl-α-D-maltoheptaosides onto a column of only 10x60 cms. without precipitation on the packing must be considered surprising.

It is a requirement of an enzyme substrate for clinical determination that the molecule is a single chemical of known structure; more specifically, in the case of this α-amylase substrate, contamination by unblocked glucoside would result in reduced shelf-life and elevated initial absorbance. Other isomers of O-thexyldimethylsilyl-p-nitrophenyl-α-D-maltoheptaoside are known to exhibit different kinetics towards the enzyme and therefore, if present, at 2% or more would interfere with the accurate determination of α-amylase in a sample.

It is well known that primary alcohols may be selectively silylated in the presence of secondary alcohols. For example, C.M. Sharts et al (Organic Prep. Proced. Int., 1986, 8, 345-52) reported the formation of t-butyldimethylsilyl or t-butyldiphenyl-silyl ether of the $C_{24}$ primary alcohol of a bile alcohol derivative, in the presence of two secondary alcohols.

P.J. Gaudemans et al (J. Org. Chem., 1987, 52, 5255-60) have selectively silylated the primary 6-hydroxyl of C1-substituted galactosides using silver nitrate-catalysis, but this neither teaches nor suggests how this could be achieved with an oligosaccharide. However, due to the expense of this reagent this is not in any case a scaleable commercial process.

F. Franke and R.D. Guthrie (Aust. J. Chem., 1977, 30, 639-47) have selectively silylated methyl-α-D-glucopyranoside at the 6-position using t-butyldimethylsilyl chloride in pyridine. When the same authors attempted to form a mono-silyl derivative of a disaccharide (sucrose), carrying out the procedure under conditions which would promote monosilylation (i.e. excess sucrose), the required derivative was produced at 10.5% in a complex mixture of products. Other components of the mixture were due to over-silylation as all three primary hydroxyl groups in the sucrose molecule were taking part in the reaction to give a variety of products. This paper does not show how the monosilylated product may be purified on a commercial scale. More importantly, it is indicated that the yield of 10.5% is what would be expected from a disaccharide almost as a mathematical chance. Thus, in the case of a heptaoside, the expected yield would be about 3%. Hence, to obtain selective silylation in excess of such a figure is surprising and to obtain a significantly higher figure of the order of 10% is remarkable.

Therefore, the exemplified cases only achieved selective silylation of a primary hydroxyl in the presence of 2 or 3 secondary hydroxyls. The present requirement was the silylation of the primary 6-hydroxyl group on the terminal non-reducing glucose unit, in order to produce a compound suitable for use as a substrate for the assay of α-amylase. In order to produce the desired product (5) directly from the unprotected starting p-nitrophenyl-α-D-maltoheptaoside (1), it was necessary to obtain selective silylation of the primary 6-hydroxyl on the terminal glucose unit in the presence of 6 other primary hydroxyls of glucose and 15 secondary hydroxyls, i.e. a total of 21 other reactive groups. Inter alia, the report by Franke and Guthrie on the silylation of sucrose would lead one to expect a very complex mixtrue of monosilylated species with a very low content of the desired monosilylated product. In order to prepare the desired product (5) from the direct combination of silylating agent and starting material (1), silylation must occur on only the 6-hydroxyl of the terminal glucose. This must result with sufficient selectivity to allow recovery of this material in good yield from a manageable mixture of other mono- and poly-silylated species.

It has now surprisingly been discovered that the primary 6-hydroxyl of the terminal glucose may be selectively silylated in the presence of the other unprotected hydroxyls on this glucose and the other 6 glucose units. By the presently-preferred procedure, a mixture of silylated products is obtained, with the required compound (5) surprisingly being the largest component.

It has also unexpectedly been found that the present process is not particularly temperature dependent. In order to improve selectivity in chemical reactions, it is usual that the temperature is reduced as far as practicably possible. In the present case, the reaction proceeds equally well at room temperature as it does at -10°C, for example. The described process may be applied to a variety of starting materials similar to compound (1) in that they are oligosaccharides having an aglycone at the reducing end of the molecule. Thus, for example, the present process may be applied to the various substrates for α-amylase. The oligosaccharide may contain other than seven glucose units, three or more would be typical. The aglycone generally comprises a group directly or indirectly suitable for use in the detection of the activity of α-amylase, in particular, such as nitrophenyl, 2-chloro-4-nitrophenyl, 4-methylumbeliferone or luciferin, as well as other chromogenic phenols.

In general terms, the presently-preferred procedure comprises the reaction of p-nitrophenyl-α-D-maltoheptaoside (1) with a suitable silylating agent in a suitable solvent in the presence of a base.

More particularly, the present invention provides a process for the direct silylation of the terminal, non-reducing glucose unit of an oligosaccharide containing at least three repeating glucose units and having an aglycone unit at the reducing end thereof, the aglycone being selected from nitrophenyl, 2-chloro-4-nitrophenyl, 4-methylumbeliferone, luciferin and chromogenic phenols, which comprises:

(a) forming a solution of the oligosaccharide in a solvent:

(b) reacting the oligosaccharide with a silylating protective agent in the presence of a base; and

(c) recovering a monosilylated oligosaccharide derivative from the reaction mixture.

Examples of silylating agents which may be used include thexyldimethyl silyl chloride, bromide, iodide and trifluoromethane sulphonate, as well as other such agents known to those skilled in the art, for example chlorotrimethylsilane, bis-trimethylsilylacetamide, chlorotriphenylsilane and chloro-t-butyl-dimethylsilane. Thexyldimethyl silyl chloride is generally preferred. Solvents, such as N, N-dimethylformamide and pyridine, or other unreactive solvents may be used. The presently-preferred solvent is pyridine. The base used may be, for example, triethylamine, diisopropylamine, pyridine or imidazole. The preferred base is generally pyridine. In this procedure, pyridine, for example, may serve as both the base and the solvent. The reaction pH should generally be greater than 7.0.

The temperature of the reaction may be from -40 to +30°C, for example, with a preferred temperature being from -15 to +5°C.

The following Examples illustrate certain preferred aspects of the present invention.

## Example 1

A 20 litre flask equipped with stirrer and thermometer was charged with pyridine (10 l) and p-nitrophenyl-$\alpha$-D-maltoheptaoside (1kg). The mixture was stirred for 16 hours to obtain a clear solution. The solution was cooled to -10°C and dimethylthexylsilyl chloride (309ml) was added slowly over 2 hours. The solution was then stirred for 16 hours, while warming to room temperature. HPLC analysis showed 16.9 % conversion to the required product with 50 % starting material present. (Column - "SPHERISORB ODS" 25 cm x 4.6 mm. Mobile phase - methanol/water gradient. Detection - UV 305 nm. Injection volume - 20 $\mu$l.) In order to consume more of the starting material, and thus obtain an improved yield of the required product, a further quantity of silyl chloride was required. Therefore, after re-cooling to -10°C, a further quantity (13 ml) of the silyl chloride was added. The solution was stirred for 16 hours while warming to room temperature. The solution was re-cooled to -5°C and a further quantity (16ml) of the silyl chloride was added. (The step of sampling and HPLC determination of the concentration of the required compound, combined with further addition of silylating agent may be repeated as necessary to maximise the yield.) After stirring for 5 hours, the reaction was quenched by the addition of methanol (100ml). Evaporation in vacuo gave 1782g of crude solid containing 200g of the required product, which represents a 20% yield based on the starting material.

## Example 2

p-nitrophenyl-$\alpha$-D-maltoheptaoside (110g) was stirred overnight in pyridine (110 ml) under nitrogen at room temperature (ca 20 °C) to give a hazy solution. The solution was cooled to -10 °C and dimethylexylsilyl chloride (25.86 ml) was added. The mixture was stirred at -10 °C for 3.75 hours and then stored at 5°C. The reaction was monitored by HPLC to determine when the optimum conversion had been achieved. After a total time of 46.25 hours, the reaction was quenched by the addition of methanol (5ml). Evaporation in vacuo of the solution gave 177.3g of crude solid containing 20g of the required monosilyated product.

## Example 3

A solution of p-nitrophenyl-$\alpha$-D-maltoheptaoside (2g) in pyridine (40ml) was cooled to -25°C. Dimethylthexyl silyl trifluoromethane sulphonate (0.8ml) was added and the mixture stirred for 16 hours, while warming to room temperature. HPLC analysis showed 15.6% conversion to the required monosilylated product.

## Example 4

A solution of p-nitrophenyl-$\alpha$-D-maltoheptaoside (5g) in N,N-dimethylformamide (50ml) and diisopropylamine (1.71ml) was cooled to -10°C. Dimethylthexylsilyl chloride (1.93ml) was added and the mixture stirred for 2 hours at -10°C and then for $19\frac{1}{2}$ hours at +5°C. HPLC analysis showed 15.4% conversion to the required monosilylated product.

## Example 5

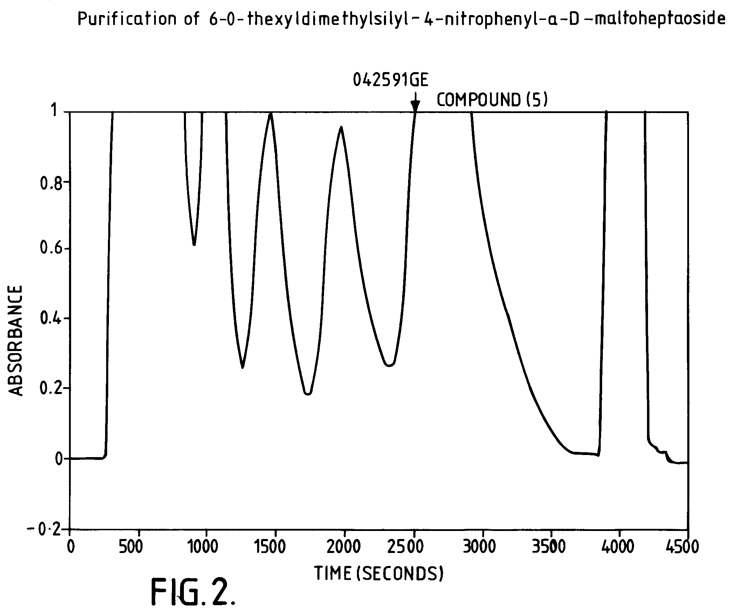

Crude product from Example 1 (300g) was mixed with methanol (0.75 l), followed by acetonitrile (75ml) and demineralised water (0.675 l) at room temperature with complete solubilisation of the 6-0-thexyldimethylsilyl-p-nitrophenyl-$\alpha$-D-maltoheptaoside. The solution (1.75 l) was loaded onto a cartridge (10 x 60cm) of $C_{18}$ derivatised silica particles (Bondapak) subject to radial compression at 500 psi (3435 Kpu). The packing material had previously been cleaned with 0.25% acetic acid in methanol:water 55:45 v/v prior to use. The separation was performed using a mobile phase of water : methanol (containing 0.1 g/l Trizma, pH 8.9) 45:55 v/v at 0.69 l/min. Product was recovered in a single peak at 75% yield, (see accompanying Figure 2). The product fraction was concentrated in vacuo at 40 °C to remove the methanol and then lyophilised at 10 °C to yield 15g of the desired product. This represents a yield from the crude product of 44.5% purified material. Analysis of the purified product by HPLC ($NH_2$ microbondapak elution with $CH_2CN/H_2O$: 72/78 (v/v) at a flow rate of 2 ml/min, detection at 313nm) indicated a purity of approximately 99.2%.

For purposes of illustration, preferred aspects of the present process have been largely described at this time with particular reference to one oligosaccharide derivative, 6-0-thexyldimethylsilyl-p-nitrophenyl-$\alpha$-D-maltoheptaoside. However, as will be appreciated by those skilled in the art, the present process may also be applied to the preparation of similar derivatives of different chain length or having non-interferring variations in structure or substituents, for example.

## Claims

**1.** A process for the direct silylation of the terminal, non-reducing glucose unit of an oligosaccharide containing at least three repeating glucose units and having an aglycone unit at the reducing end thereof, the aglycone being selected from nitrophenyl, 2-chloro-4-nitrophenyl, 4-methylumbeliferone, luciferin and chromogenic phenols, which comprises:

(a) forming a solution of the oligosaccharide in a solvent:

(b) reacting the oligosaccharide with a silylating protective agent in the presence of a base; and

(c) recovering a monosilylated oligosaccharide derivative from the reaction mixture.

**2.** A process as claimed in claim 1 wherein the oligosaccharide is p-nitrophenyl-α-D-maltoheptaoside.

**3.** A process as claimed in claim 1 or claim 2 wherein the monosilylated oligosaccharide derivative is 6-0-thexyldimethylsilyl-p-nitrophenyl-α-D-maltoheptaoside.

**4.** A process as claimed in any of claims 1 to 3 wherein the silylating protective agent is selected from thexyldimethyl silyl chloride, bromide, iodide and trifluoromethane sulphonate, chlorotrimethylsilane, bis-trimethylsilylacetamide, chlorotriphenylsilane and chloro-t-butyl-dimethylsilane; preferably thexyldimethyl silyl chloride.

**5.** A process as claimed in any of claims 1 to 4 wherein the solvent is N,N-dimethylformamide or, preferably, pyridine.

**6.** A process as claimed in any of claims 1 to 5 wherein the base is selected from triethylamine, disopropylamine, imidazole and pyridine; preferably pyridine.

**7.** A process as claimed in any of claims 1 to 6 wherein pyridine serves as both the solvent and the base.

**8.** A process as claimed in any of claims 1 to 7 wherein the reaction temperature is from -40 to +30°C, preferably from -15 to +5°C.

**9.** A process as claimed in any of claims 1 to 8 wherein the monosilylated oligosaccharide derivative is recovered by HPLC.

FIG.1.

Purification of 6-0-thexyldimethylsilyl-4-nitrophenyl-α-D-maltoheptaoside

FIG. 2.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | JOURNAL OF ORGANIC CHEMISTRY. vol. 52, no. 23, 1987, EASTON US pages 5255 - 60; E.M.NASHED ET AL.: 'Selective Silylation of Beta-D-Galactosides. A New Approach to the Synthesis of (1->6)-Beta-D-Galactopyranooligosaccharides.' | 1 | C07H23/00 C12Q1/40 C07H15/203 C07H3/06 |
| D,A | EP-A-0 135 758 (BOEHRINGER MANNHEIM GMBH) * the whole document * | 1 | |
| D,A | EP-A-0 171 960 (GENZYME CORPORATION) * the whole document * | 1 | |
| P,A D | EP-A-0 452 067 (GENZYME CORPORATION) * the whole document * | 1 | |
| D,A | AUSTRALIAN JOURNAL OF CHEMISTRY vol. 30, 1977, EAST MELBOURNE AU pages 639 - 47; F.FRANKE ET AL.: 't-Butyldimethylsilyl Ethers of Sucrose.' * the whole document * | 1 | TECHNICAL FIELDS SEARCHED (Int. Cl.5 ) C07H C12Q |

The present search report has been drawn up for all claims

| Place of search | Date of completion of the search | Examiner |
|---|---|---|
| THE HAGUE | 17 SEPTEMBER 1992 | SCOTT J.R. |